# EUROPEAN PATENT SPECIFICATION

(11) **EP 1 210 043 B1**
(45) Date of publication and mention of the grant of the patent: **23.07.2003**
(21) Application number: 99954353.1
(22) Date of filing: 26.10.1999
(51) Int. Cl.: A61F 9/01

(54) **APPARATUS FOR DETERMINING AND ABLATING THE CORNEAL TISSUE VOLUME FOR CORRECTING VISUAL AMETROPIA**
GERÄT FÜR DAS ERFASSEN UND ABTRAGEN VON HORNHAUTGEWEBE ZUM KORRIGIEREN DER AMETROPIE
APPAREIL POUR CALCULER ET RETIRER LE VOLUME DE TISSU CORNEEN NECESSAIRE POUR CORRIGER L'AMETROPIE VISUELLE

(30) Priority: 12.07.1999 IT MI991526
(43) Date of publication of application: 05.06.2002
(60) Divisional application: 03009955.0
(73) Proprietor: Ligi Tecnologie Medicali S.p.A., 74100 Taranto (IT)
(72) Inventor: D'IPPOLITO, Giuseppe, 74100 Taranto (IT)
(74) Representative: Jorio, Paolo
(86) International application number: IT9900341
(87) International publication number: WO01003621

(56) References cited:
- EP-A- 0 568 081
- WO-A-92/01430
- US-A- 4 721 379
- US-A- 5 455 766
- US-A- 5 490 098
- US-A- 5 891 132

## Description

### BACKGROUND OF THE INVENTION

The present invention relates to an apparatus for determining and ablating the corneal tissue volume necessary for correcting visual ametropia, as optimized for each individual patient.

As is known, for correcting visual ametropia, it is necessary to perform a corneal tissue ablation, and optimize it depending on the clinic status of each individual patient.

The document US-A-5 891 132 relates to a system for controlling excimer laser eye surpery substantially according to the preamble of claim 1.

### SUMMARY OF THE INVENTION

The aim of the present invention is to provide an apparatus adapted to mutually coordinate a set of operating devices, so as to univocally define the position, area and volume of the corneal structure to be ablated, for consequently performing the related processing.

Within the scope of the above mentioned aim, a main object of the present invention is to provide such an apparatus which is very efficient from an operating standpoint and which is specifically designed for providing accurate values to properly perform the ablating operation.

Yet another object of the present invention is to provide such an apparatus which, owing to its constructional peculiar features, is very reliable and safe in operation.

Yet another object of the present invention is to provide such an apparatus which can be easily made starting from easily available elements and materials and which, moreover, is very competitive from a mere economic standpoint.

According to one aspect of the present invention, the above mentioned aim and objects, as well as yet other objects, which will become more apparent hereinafter are achieved by an apparatus for determining and ablating a corneal tissue volume necessary for correcting visual ametropia, as optimized for each individual patient, said apparatus comprises a central control unit, to which are operatively connected, a corneal topograph, an infrared pupillometer, a scansion laser and an excimer or solid-state laser as defined in claim 1.

### BRIEF DESCRIPTION OF THE DRAWINGS

Further characteristics and advantages of the present invention will become more apparent hereinafter from the following detailed disclosure of a preferred, though not exclusive, embodiment of an apparatus for determining and ablating an optimum corneal tissue volume necessary for correcting visual ametropia, and being illustrated, by way of an indicative, but not limitative example, in the accompanying drawings, where:
Figure 1 illustrates an operating flow diagram, associated with the apparatus according to the present invention; and
Figure 2 illustrates an operating block diagram of the apparatus according to the present invention.

### DESCRIPTION OF THE PREFERRED EMBODIMENTS

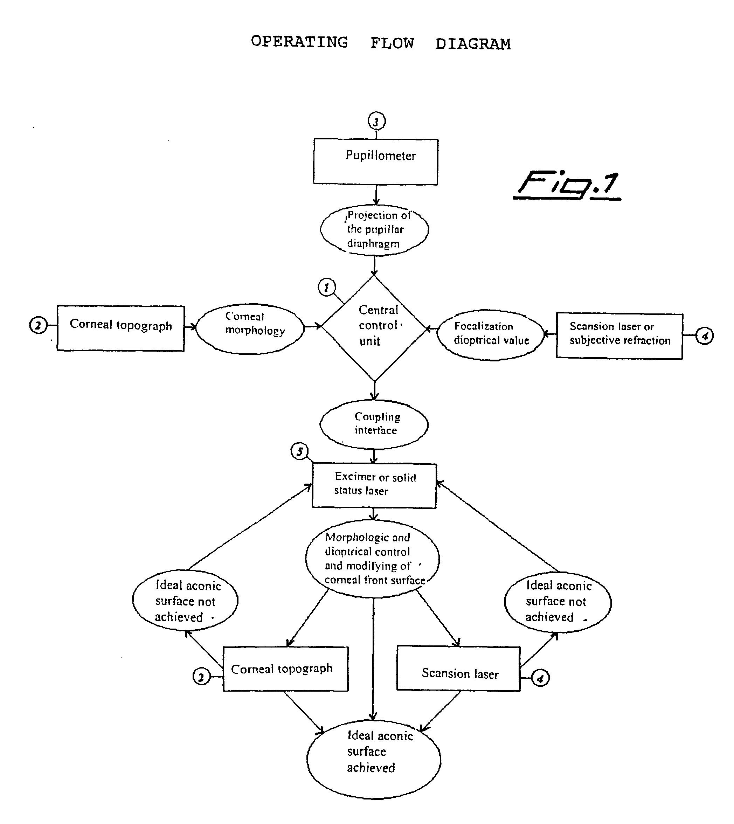

With reference to the number references of Figure 1, the apparatus for determining and ablating the corneal tissue volume necessary for correcting visual ametropia, as optimized for each individual patient, comprises a central control unit, generally indicated by the reference number 1, to which is connected a corneal topograph 2, operating for morphologically determining the front surface of the cornea.

To said unit 1 is connected an infrared pupillometer 3, for determining the iris diaphragm projection under scotopic conditions, at the level of the front corneal surface.

To said central unit is moreover connected a scansion laser 4 operating to define the dioptrical corrective value to be applied, point by point, with a discrete type of function, to the front corneal surface.

This value is determined by searching the diopter value optimizing the focalization at foveal level of each single light beam impinging on the corneal region, as in turn generated by a light source arranged at an infinite distance.

To said control unit 1 is moreover connected an excimer or solid state laser, of a microspot type, 5, having a coupling interface, for reading-out the altimetric ablative datum as expressed in micrometers on a square matrix in the x, y plane.

The apparatus according to the present invention allows to determine the ablating volume obtained by a crossing of the front corneal surface and an ideal aconic surface, as determined at a level of the pupillar diaphragm projection, as detected in a scotopic condition.

The front corneal surface is given by the corneal topograph 2, whereas the scansion laser 4, which constitutes the main feature of the present invention, measures point by point, with a discrete type of function, the angular deviation of the light beam impinging the cornea, which source is at infinite distance, to match the projection of said light beam on the retina with the fovea or center of the vision.

Once the angular deviation it is measured, by means of the biunivocal correspondence between impinging beam and refracted beam, the ideal slopes of the cornea are calculated to match all of the projections of the light beams onto the fovea or center of the vision.

The ideal aconic surface for the cornea is the one which minimize the sum or summation function, as expressed in absolute value, of the differences between the ideal slopes measured with the scansion laser 4 and the slopes of the ideal aconic surface or, in other words, the one which minimize the summation function, as expressed in absolute value, of the eccentricity, as measured in a radial direction, between the projection of the light beam impinging on the retina and the fovea or center of vision.

The sum or summation function analyzes, by discrete steps, all of the points where the impinging light beams cross the corneal front surface at an area delimited by the projection of the pupillar diaphragm as detected in a scotopic condition.

The connecting area between the ideal aconic surface and the residual corneal front surface is determined by assuring a constant curvature variation in a radial direction.

During the practical operation of the apparatus, an operator will detect, at the start, the corneal front surface by using said topograph 2.

Then, the operator will define the localization of said surface by locating the processing center as well as the reference axis.

The processing center, in particular, can be constituted by the corneal apex, the pupillar centroid projection, the fixation reflex or it can be selected at will by the operator.

The reference axis can comprise the optical axis, or the TV camera axis, or an axis perpendicular to the processing center.

The optical zone is delimited by the crossing perimeter of the corneal front surface, as determined by the topograph and localized as stated, and the preset ideal aconic surface.

The altimetric dislocation or offset between the two mentioned surfaces will be defined by determining the specific crossing perimeter which is circumscribed to the diameter referred to a projection on the corneal front surface of the pupillar diaphragm as detected in a scotopic condition and by the infrared pupillometer 3.

This function assures that the processing be performed through the overall corneal portion affected by the refractive process, by specifically limiting the overall volume to be ablated to a minimum necessary volume.

As the crossing perimeter exceeds the maximum diameter specified by the operator, it will be limited, for the exceeding portion, to the mentioned diameter.

Alternatively, said ideal aconic surface can be determined by a subjective refraction of the patient, as expressed in terms of correcting dioptrical values, either spherical and/or cylindrical, with the related asphericity axis and index.

In such a case, it is necessary to define the reference corneal refraction by determining that aconical surface adapted to better approximate the patient corneal front surface the center, axis and optical zone thereof being specified as previously stated.

The curvature, either maximum or minimum, of the preset aconical surface, referred to the corneal front surface refraction index, will express the related refractive power, in the term of the maximum and minimum values thereof.

That same aconical surface will express, moreover, the reference asphericity index.

In this latter case, the ideal aconic surface will be obtained from a vectorial summation of the refraction related to the aconic surface better approximating the corneal front surface, as determined as previously stated, and of the subjective refraction, as expressed as previously stated.

In particular, the operator pre-selects the surgical mode of operation, by choosing between PRK or LASIK, in order to properly correlate the processing amount and size, as designed at the level of the corneal front surface, depending on the actual distance thereof from the focal plane.

In particular, the operator preselects the target minimum width for the transition zone and the optional maximum ablation additional thickness, to obtain a constant curvature variation in a radial direction through the transition area, connecting the desired ideal aconic surface and residual corneal front surface.

Then, the operator will moreover define a maximum diameter for limiting therewithin the perimeter of the transition area.

The operator, in the case of a surgical PRK mode of operation, can possibly define, to obtain a laser corneal processing, the area thereon to add a constant thickness to be ablated, as defined with reference to the total processing perimeter, as well as the related value.

The above defined mode of operation, will establish univocally the processing mode both at a planimetric and at an altimetric level.

It has been graphically represented by different color areas, clearly showing the ideal aconic surface, the transition area between the ideal aconic surface and the residual corneal front surface, as well as the residual corneal front surface.

Moreover, by numeric data the operator will express or define the total ablation surface, the total ablation volume, the maximum ablation thickness and related planimetric dislocation as well as the ablation center planimetric dislocation with respect to the pupillar centroid.

The altimetric ablation datum, in particular, will be defined on a square matrix in the x, y plane, to allow the laser 5 to detect, through a suitable interface, the ablation profile or contour to be consequently performed.

As desired, the system, by intraoperatively detecting the morphologic datum and dioptrical variation will verify that the processing be carried out in accordance with the programmed ablation schedule, by modifying the latter, if necessary, together with the number of pulses localized for surface unit.

The processing is ended after having obtained a proper fitting of the detected and desired data.

From the above disclosure it should be apparent that the invention fully achieves the intended aim and objects.

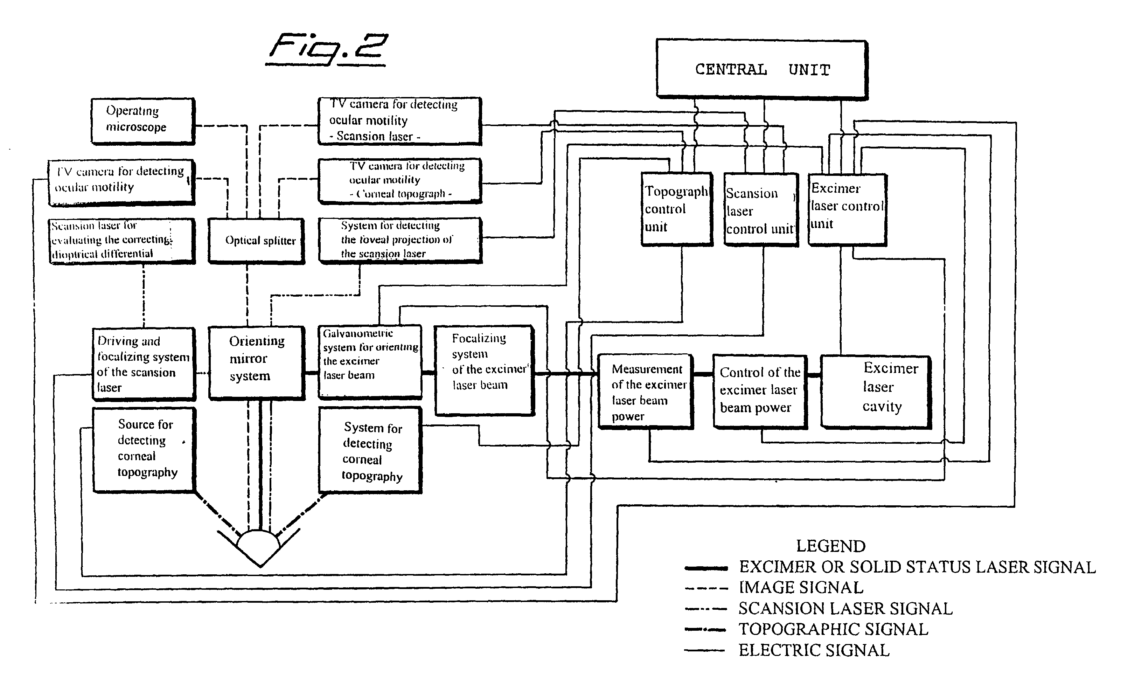

In particular, an apparatus according to the functional block diagram shown in Figure 2 has been provided, which apparatus is specifically adapted to provide very accurate and objective elements for controlling and performing the surgical operation.

## Claims

1. An apparatus for determining and ablating a corneal tissue volume of an eye of a patient necessary for correcting visual ametropia of said eye, comprising a central control unit (1), a corneal topograph (2) for morphologically defining a corneal front surface, an infrared pupillometer (3) for measuring a diameter of a pupil of said eye and an excimer or solid-state laser (5) all of which are respectively operatively connected to said central control unit (1), **characterized in that** said apparatus further comprises a scansion laser (4) coupled to said central unit (1), said scansion laser being adapted to measure, point by point, an angular deviation of a light beam impinging on the eye cornea to match the projection of said light beam on the retina with the fovea or center of the vision to define a correcting dioptrical value to be applied to said corneal front surface to optimize a focalization, at foveal level, of the light beam source, arranged at an infinite distance, the impinging light beams of said light source crossing the corneal front surface, through an area of the pupillar diaphragm projection as detected under scotopic conditions.

2. An apparatus, according to Claim 1, chacterized in that said infrared pupillometer (3) is adapted to define an iris diaphragm projection, under scotopic conditions, at a level of said corneal front surface.

3. An apparatus, according to Claims 1 and 2, **characterized in that** said excimer or solid state laser (5) is of a microspot type, with a coupling interface for coupling said excimer laser (5) to said central control unit (9), and that said interface is adapted to read-out an altimetric ablation datum as expressed in micrometers on a square matrix in an x, y plane.

## Patentansprüche

1. Vorrichtung zum Bestimmen und Abtragen des zum Korrigieren der visuellen Ametropie notwendigen Cornea-Volumens eines Auges eines Patienten mit
einer zentralen Steuereinheit (1);
einem Cornea-Tophograph (2) zum morphologischen Bestimmen einer vorderen Cornea-Oberfläche;
einem Infrarotpupillometer (3) zum Messen eines Durchmessers einer Pupille von dem Auge; und
ein Excimer oder Festkörperlaser (5), wobei alle operativ mit der zentralen Steuereinheit (1) verbunden sind;
dadurch charakterisiert, daß
die Vorrichtung einen mit der zentralen Steuereinheit (1) gekoppelten Abtastungslaser (4) aufweist, wobei
der Abtastungslaser (4) angepaßt ist, eine Winkelabweichung eines auf der Augen-Cornea auftreffenden Lichtstrahls Punkt für Punkt zu messen, um die Projektion des Lichtstrahls auf die Retina mit der Fovea oder des Sehzentrums anzupassen, um einen korrigierenden Dioptrienwert zu definieren, der ein für die vordere Cornea-Oberfläche anzuwendenden Wert ist, um eine Fokussierung der in einem unendlichen Abstand angeordneten Lichtstrahlquelle auf fovealen Ebene zu optimieren, wobei
die auftreffenden Lichtstrahlen der Lichtquelle die vordere Cornea-Oberfläche durch einen Bereich der unter scotopischen Bedingungen erfaßten Pupillenblendenprojektion kreuzen.

2. Vorrichtung nach Anspruch 1, **dadurch gekennzeichnet, daß** das Infrarotpupillometer (3) angepaßt ist, eine Irisblendenprojektion unter scotopischen Bedingungen zu einer Ebene der vorderen Cornea-Oberfläche zu definieren.

3. Vorrichtung nach Anspruch 1 oder 2, **dadurch gekennzeichnet, daß** der Excimer oder Festkörperlaser (5) vom microspot Typ mit einer Koppelschnittstelle zum Ankoppeln des Excimer-Laser (5) an die zentrale Steuereinheit (1) ist, und daß die Schnittstelle zum Auslesen eines in Mikrometern auf einer quadratische Matrix in einer x, y Ebene ausgedrückten altrimetrischen Abtragungsbezugspunkts angepaßt ist.

## Revendications

1. Un appareillage pour la détermination et l'ablation d'un volume de tissu cornéen d'un oeil d'un patient, nécessaire pour la correction de l'amétropie visuelle dudit oeil, comprenant une unité centrale de contrôle (1), un topographe cornéen (2) pour définir morphologiquement une surface frontale cornéenne, un pupillomètre à infrarouge (3) pour mesurer un diamètre d'une pupille dudit oeil et un laser à excimère ou à état solide (5), l'ensemble étant respectivement connecté de manière opérationnelle à ladite unité centrale de contrôle (1), **caractérisé en ce que** ledit appareillage comprend au surplus un laser à scansion (4) couplé à ladite unité centrale (1), ledit laser à scansion étant adapté pour mesurer point par point une déviation angulaire d'un faisceau lumineux frappant la cornée de l'oeil de manière à concorder avec la projection dudit faisceau lumineux sur la rétine avec la fovéa ou le centre de la vision de manière à définir une valeur dioptrique de correction destinée à être appliquée à ladite surface frontale cornéenne pour optimiser une focalisation au niveau fovéal de la source du faisceau lumineux, située à une distance infinie, les faisceaux lumineux incidents de ladite source de lumière traversant la surface frontale cornéenne à travers une zone de la projection du diaphragme pupillaire telle que détectée en conditions scotopiques.

2. Un appareillage selon la revendication 1, **caractérisé en ce que** ledit pupillomètre à infrarouges (3) est adapté pour définir une projection du diaphragme de l'iris en conditions scotopiques, au niveau de ladite surface frontale cornéenne.

3. Un appareillage selon les revendications 1 et 2, **caractérisé en ce que** ledit laser à excimère ou à l'état solide (5) est du type à microspot, avec une interface de couplage en vue de coupler ledit laser à excimère (5) à ladite unité centrale de contrôle (9) et **en ce que** ladite interface est adaptée pour lire un résultat d'ablation altimétrique tel qu'exprimé en micromètres sur une matrice carrée dans un plan x, y.
